# EUROPÄISCHE PATENTSCHRIFT

(11) **EP 1 400 517 B1**
(45) Veröffentlichungstag und Bekanntmachung des Hinweises auf die Patenterteilung: **10.01.2007**
(21) Anmeldenummer: 03019621.6
(22) Anmeldetag: 04.09.2003
(51) Int. Cl.: C07D 235/18, A61Q 17/04

(54) **Verfahren zur Herstellung von 2-Phenyl-benzimidazol-5-sulfonsäure aus 3,4-Diaminobenzol-sulfonsäure**
process for the preparation of 2-Phenyl-1H-benzimidazole-5-sulfonic acid from 3,4-Diaminobenzenesulfonacid
Procédé de préparation de l'acide 2-phenyl-benzimidazole-5-sulfonique à partir de l'acide 3,4-diamino-benzosulfonique

(30) Priorität: 17.09.2002 DE 10243027
(43) Veröffentlichungstag der Anmeldung: 24.03.2004
(73) Patentinhaber: Saltigo GmbH, 51369 Leverkusen (DE)
(72) Erfinder: Rauchschwalbe, Günter, Dr., 51375 Leverkusen (DE); Emde, Herbert, Dr., 51109 Köln (DE); Kissener, Wolfram, Dr., 53819 Neunkirchen-Seelscheid (DE); Paetz, Klaus-Christian, Dr., 51399 Burscheid (DE)
(74) Vertreter: Wichmann, Birgid

(56) Entgegenhaltungen:
- EP-A- 1 167 359
- WO-A-98/06703
- WO-A-03/032984
- DE-C- 4 203 072
- FR-A- 1 522 040

## Beschreibung

2-Phenyl-benzimidazol-5-sulfonsäure bzw. ihr Na-Salz ist ein wichtiges Hautschutzmittel, das Sonnenschutzmitteln zugesetzt wird, um ultraviolettes Licht bei ca. 280-320 nm ("UV-B-Strahlen") zu absorbieren. Das ist aus der DE-A 676 103 bekannt.

Das Produkt ist z.B. unter dem Namen NeoHeliopan Hydro® der Haarmann&Reimer GmbH seit langer Zeit im Handel und dem Fachmann wohlbekannt. Der einschlägige Stand der Technik zu diesem Produkt findet sich z.B. in der EP-A 669 323.

Zur Synthese wurde vorgeschlagen, 1,2-Diaminobenzol, Benzoesäure oder Derivate der Benzoesäure (wie Benzoesäureester oder Benzonitril) und Schwefelsäure zusammen zu erhitzen. Jedoch erreicht die Ausbeute an reiner Ware nur 49 bis 60 % (siehe DE 4 203 072).

Das Verfahren ist im technischen Maßstab nur schwierig durchzuführen, da bei den erforderlichen Reaktionsbedingungen Benzoesäure aus dem Ansatz heraus sublimiert und die Abgasleitungen verstopft.

Wird 1,2-Diaminobenzol unter saurer Katalyse mit Benzoesäure-alkylester umgesetzt, so bilden sich N-alkylierte Verbindungen.

Eine bekannte Methode besteht darin, 1,2-Diaminobenzol mit Benzoesäure in Anwesenheit von Polyphosphorsäure zum Benzimidazol zu kondensieren und dieses z.B. mit Chlorsulfonsäure zu sulfieren (Am. Chem. Soc. 79, 427 (1957)).

Dieses Verfahren ist industriell kaum zu realisieren, da der technische Umgang mit der sehr zähen und teuren Polyphosphorsäure umständlich und nicht wirtschaftlich ist und dabei große Mengen an Phosphorsäure in die Vorfluter geraten und diesen eutrophieren können.

Eine Alternative besteht darin, 1,2-Diaminobenzol mit Benzaldehyd in Anwesenheit von schwefliger Säure zum 2-Phenyl-benzimidazol zu kondensieren und dann weiter wie beschrieben zu sulfieren; es ist jedoch bekannt, dass sich unter diesen Umständen 1-Benzyl-2-phenyl-benzimidazol als unerwünschtes Nebenprodukt bildet, das nur schwer abzutrennen ist.

Der Sulfierschritt ist ebenfalls problematisch, da sich u.a. Nebenkomponenten auch eine isomere Sulfonsäure bildet, die nicht oder nur mit großem Aufwand abgetrennt werden kann. Außerdem wird das Produkt aus dieser Synthese häufig in verfärbter Form erhalten, so dass das Produkt für den gewünschten kosmetischen Anwendungsbereich nicht eignet.

In der FR-A-1522040 wird die Kondensation von Diaminobenzol mit Benzaldehyd in Gegenwart eines Oxidationsagens durchgeführt, wobei das Diaminobenzol mit einem Sulfonsäureester substituiert sein kann.

Es besteht daher immer noch ein Bedarf nach einem Verfahren, nach dem 2-Phenylbenzimidazolsulfonsäure in hoher Reinheit und hoher Ausbeute und gleichzeitig auf einfach durchführbare Weise hergestellt werden kann. Es bestand daher die Aufgabe, ein neues Verfahren zur Herstellung von 2-Phenylbenzimidazolsulfonsäure zur Verfügung zu stellen.

Es wurde nun überraschenderweise gefunden, dass isolierte 3,4-Diaminobenzolsulfonsäure mit Benzaldehyd und schwefliger Säure zu 2-Phenyl-benzimidazol-5-sulfonsäure umgesetzt werden kann, die dabei in ausgezeichneter Ausbeute und hoher Reinheit erhalten wird. Diese Ware ist gegebenenfalls nach einmaligem Umlösen aus wässrigem Milieu so rein, besonders ist sie strahlend weiß, dass sie für kosmetische Zwecke verwendet werden kann.

Das war auf dem geschilderten Stand der Technik nicht vorherzusehen.

Gegenstand der Erfindung ist daher ein Verfahren zur Herstellung von 2-Phenylbenzimidazol-5-sulfonsäure, dadurch gekennzeichnet, dass man 3,4-Diaminobenzolsulfonsäure bei einem pH-Wert zwischen 4 und 7 in wässriger Lösung mit 0,9 bis 1,5 mol Benzaldehyd pro Mol 3,4-Diaminobenzolsulfonsäure und 1,0 bis 3,0 mol SO₂ pro Mol 3,4-Diaminobenzolsulfonsäure oder einem Agens, das 1,0 bis 3,0 Mol SO₂ pro Mol Diaminobenzolsulfonsäure enthält, umsetzt und gegebenenfalls durch Zugabe eines oxidierenden Agens das erhaltene Produkt reinigt.

Die Herstellung von 3,4-Diaminobenzolsulfonsäure durch Sulfierung von 1,2-Diaminobenzol erlaubt die Produktion großer Mengen dieses Vorproduktes und damit den hier geschilderten technischen Fortschritt.

Im erfindungsgemäßen Verfahren gibt man in einer bevorzugten Ausführungsform zu einer wässrigen Suspension von 3,4-Diaminobenzol-sulfonsäure zunächst soviel Base, dass die eventuell anhaftende Schwefelsäure ebenso neutralisiert wird wie die Sulfonsäure. Als Base eignen sich LiOH, NaOH, KOH, Lithium-, Natrium- bzw. Kaliumcarbonat oder -bicarbonat, MgO, MgCO₃, aber auch organische Basen wie Trialkylamin oder Pyridin können stöchiometrisch oder als Zusatz von wenigen Prozenten und im Gemisch mit anderen Basen verwendet werden.

NaOH bzw. KOH sind bevorzugte Basen.

Insgesamt richtet sich die Gesamtmenge der Base nach der Menge an anhaftender und enthaltener Säure; es soll sich ein pH-Wert zwischen 7 und 4 einstellen, bevorzugt wird ein Bereich von 6 bis 5.

Die eingesetzte Wassermenge ist ebenfalls nicht kritisch; sie wird nach Rührbarkeit einerseits und möglichst hoher Konzentration zur Erreichung hoher Raumausbeute andererseits gewählt.

Charakteristisch ist eine Wassermenge von 0,5 bis 3 Ltr. , bevorzugt von 1-1,5 Ltr. pro Mol Edukt.

Dann gibt man als SO₂ enthaltendes Agens schweflige Säure zu, bevorzugt in Form ihrer Alkalisalze, z.B. NaHSO₃, Na₂S₂O₅ oder Na₂SO₃, doch kann SO₂ auch als solches gasförmig zudosiert und in situ mit Basen umgesetzt werden.

Die Rolle des Sulfites ist eine doppelte:

Einerseits wird der Benzaldehyd, der als Reaktionskomponente dient, unter intermediärer Bildung von Sulfit-Addukten in eine wasserlösliche Form überführt, so dass die Reaktion in homogenem Medium erfolgt; andererseits dient Sulfit als Oxidationsmittel für primär gebildete 2-Phenylbenzimidazolin-Sulfonsäure, die dann in die gewünschte Phenylbenzimidazol-sulfonsäure übergeht.

Als Salze dienen bevorzugt NaHSO₃ bzw. Na₂S₂O₅ oder Na₂SO₃. Sie werden ebenso wie ggf. SO₂ in einer Menge zugesetzt, die einer SO₂-Menge von 1 bis 3 Mol pro Mol Diaminbenzolsulfonsäure entspricht; bevorzugt werden Mengen im Bereich von 1,1 bis 2,5 Mol, besonders im Bereich von 1,2 bis 2,2 Mol/Mol.

Schließlich gibt man Benzaldehyd in ungefähr stöchiometrischer Menge zu.

Bei zu niedrigem molarem Verhältnis sinkt die Ausbeute, bei Überschuss an Benzaldehyd kann es zur Belastung des Abwassers mit diesem Überschuss kommen. I.a. arbeitet man mit einem molaren Verhältnis von 0,85 bis 1,5, bevorzugt von 0,90 bis 1,1.

Das Verfahren wird bei Temperaturen durchgeführt, die zwischen 25 °C und 130°C liegen; bevorzugt in einem Bereich von 50 bis 90°C, besonders bevorzugt 60 bis 80°C. Die Durchführung ist nicht besonders empfindlich gegen Temperaturvariationen; die Temperatur darf nicht zu niedrig sein, da die Reaktion sonst zu langsam abläuft; bei einer Verfahrenstemperatur von deutlich über 100° ist die Verwendung einer Druckapparatur erforderlich.

Die im gewählten Bereich erforderliche Reaktionszeit ist vom Fachmann sehr leicht festzustellen, z.B. mit Hilfe von IR-Spektroskopie, HPLC, Dünnschichtchromatografie oder ähnlichen analytischen Methoden. Im Bereich von 60-80°C ist die Reaktion im allgemeinen im Bereich von ½-2 Stunden durchführbar.

Kleine Mengen von Feststoffen und/oder Trübungen können mit dem Fachmann bekannten Methoden beseitigt werden, z.B. durch Behandeln mit Aktivkohle, Kieselgel, Cellulosepulver, Kaolin oder ähnlichen Hilfsmitteln, die nach der Behandlung abgetrennt werden, z.B. durch Filtrieren, Zentrifugieren, Membranpermeation und ähnlichen Verfahren.

Bevorzugt ist die Verwendung von Adsorbentien, die auch verfärbende Nebenkomponenten aus der Lösung entfernen; besonders geeignet sind verschiedene handelsübliche Aktivkohle-Sorten wie Norit.

Die klar filtrierte Lösung wird dann mit Säure angesäuert. Dazu kommt Schwefelsäure in Frage ebenso wie Salzsäure oder Essigsäure. Die Verwendung von Essigsäure hat sich besonders bewährt.

Das gewünschte Produkt fällt dabei aus und kann durch geeignete Temperaturführung und/oder Zugabe von Kristallkeimen in eine besonders gut filtrierbare Form überführt werden. Methoden dieser Art sind dem Fachmann bekannt.

Schließlich wird das Produkt durch Filtrieren, Zentrifugieren o.ä. Methoden isoliert, gewaschen (um Salze zu entfernen) und entweder getrocknet oder feucht weiterbearbeitet.

Genügt die erhaltene Reinheit, besonders der optische Aspekt, noch nicht den Anforderungen, so kann das Produkt erneut alkalisch gelöst und gegebenenfalls mit kleinen Mengen an Oxidationsmitteln behandelt werden. Als brauchbare Oxidationsmittel seien beispielhaft genannt: KMnO₄; FeCl₃; Chlorlauge; Wasserstoffperoxyd, auch in Form von Addukten mit Harnstoff oder Natriumborat; K₂S₂O₈; aktivierter Sauerstoff.

Kaliumpermanganat ist besonders bevorzugt. Es wird charakteristischerweise in einer Menge von 0,5 bis 3 g pro Mol eingesetzt, bevorzugt in einer Menge von 1-2 g pro Mol.

Bevorzugt wird nach Durchführung der Reaktion das Produkt zwischenisoliert und gelöst; dann gibt man das Oxidationsmittel zu, klärt noch einmal mit einem der o.g. Adsorbentien, trennt ab und fällt dann das nachgereinigte Produkt durch Ansäuern wieder aus.

Bei Verwendung von hinreichend reiner 3,4-Diaminobenzol-sulfonsäure kann jedoch meistens auf den Zusatz von Oxidationsmitteln verzichtet werden.

Bei Verwendung der o.g. 1,2-Diaminobenzolsulfonsäure ist diese Arbeitsweise ausreichend, um ein Produkt zu erhalten, das die meisten Ansprüche erfüllt.

Im Vergleich dazu ergibt eine 1,2-Diaminobenzolsulfonsäurelösung, die durch Hydrieren von 2-Nitro-anilin-4-Sulfonsäure an Raney-Nickel oder Pd/C in wässriger Lösung erhalten und aus der die Sulfonsäure nicht zwischenisoliert wird, ein Rohprodukt von 2-Phenylbenzimidazol-5-sulfonsäure, das wenigstens dreimal, meistens 4- bis 5-mal umgefällt werden muss, um die von der kosmetischen Industrie gestellten Reinheitsanforderungen, speziell nach reinweißem Aspekt, zu erfüllen.

### Beispiel 1

Man legt 1250 ml Wasser vor und trägt 1,0 Mol 3,4-Diaminobenzol-sulfonsäure ein (z.B. ca. 50 %ig, schwefelsäure-feucht; hergestellt aus 1,2-Diaminobenzol und Schwefelsäure).

Man tropft soviel NaOH (45-50 %ig) zu, dass sich eine klare Lösung bildet und der pH-Wert auf 5,5 eingestellt ist. Man gibt 200 g Na₂S₂O₅ zu, erwärmt auf 60°C und tropft allmählich 116 g (1,08 Mol) Benzaldehyd zu. Man erwärmt auf 80°, rührt 1 Stunde, klärt mit 8 g Aktivkohle und säuert nach dem Abfiltrieren der Aktivkohle mit Säure (z.B. mit Essigsäure) bei 80°C an.

Man rührt kalt, saugt ab und wäscht mit Wasser.

Man erhält 320 g Feuchtware und daraus 245g Trockenware (Ausbeute: 89,3 %).

Diese Ware ist bereits für die meisten Verwendungszwecke hinreichend rein.

Wird eine besonders reine und besonders weiße Ware gewünscht, so kann diese Ware noch einmal in verdünnter NaOH gelöst (ggf. ohne Zwischentrocknung), unter Zusatz von einigen Gramm Kaliumpermanganat mit Aktivkohle erhitzt und geklärt und erneut durch Ansäuern gefällt werden.

Man erhält dann 240 g Trockenware (Ausbeute: 98,0 %).

## Patentansprüche

1. Verfahren zur Herstellung von 2-Phenylbenzimidazol-5-sulfonsäure, **dadurch gekennzeichnet, dass** man 3,4-Diaminobenzolsulfonsäure bei einem pH-Wert zwischen 4 und 7 in wässriger Lösung mit 0,9 bis 1,5 Mol Benzaldehyd pro Mol 3,4-Diaminobenzolsulfonsäure und mit 1,0 bis 3,0 Mol SO₂ pro Mol 3,4-Diaminobenzolsulfonsäure, oder einem Agens, das 1,0 bis 3,0 Mol SO₂ pro Mol 3,4-Diaminobenzolsulfonsäure enthält, umsetzt.

2. Verfahren nach Anspruch 1, **dadurch gekennzeichnet, dass** man durch Sulfierung von 1,2-Diaminobenzol erhaltene 3,4-Diaminobenzolsulfonsäure einsetzt.

3. Verfahren nach einem der Ansprüche 1 bis 2, **dadurch gekennzeichnet, dass** die Reaktion bei einem pH-Wert im Bereich von 5 bis 7 durchgeführt wird.

4. Verfahren nach einem der Ansprüche 1 bis 3, **dadurch gekennzeichnet, dass** Benzaldehyd in einer Menge von 1,05 bis 1,1 mol/mol eingesetzt wird.

5. Verfahren nach einem der Ansprüche 1 bis 4, **dadurch gekennzeichnet, dass** die Reaktion bei einer Temperatur im Bereich von 25° bis 130°C, durchgeführt wird.

## Claims

1. Process for the preparation of 2-phenylbenzimidazole-5-sulphonic acid, **characterized in that** 3,4-diaminobenzenesulphonic acid is reacted at a pH between 4 and 7 in aqueous solution with 0.9 to 1.5 mol of benzaldehyde per mole of 3,4-diaminobenzenesulphonic acid and with 1.0 to 3.0 mol of SO₂ per mole of 3,4-diaminobenzenesulphonic acid, or an agent which comprises 1.0 to 3.0 mol of SO₂ per mole of 3,4-diaminobenzenesulphonic acid.

2. Process according to Claim 1, **characterized in that** 3,4-diaminobenzenesulphonic acid obtained by sulphonation of 1,2-diaminobenzene is used.

3. Process according to one of Claims 1 to 2, **characterized in that** the reaction is carried out at a pH in the range from 5 to 7.

4. Process according to one of Claims 1 to 3, **characterized in that** the benzaldehyde is used in an amount of from 1.05 to 1.1 mol/mol.

5. Process according to one of Claims 1 to 4, **characterized in that** the reaction is carried out at a temperature in the range from 25° to 130°C.

## Revendications

1. Procédé de préparation de l'acide 2-phénylbenzimidazole-5-sulfonique, **caractérisé en ce que** l'on fait réagir de l'acide 3,4-diaminobenzènesulfonique à un pH compris entre 4 et 7, en solution aqueuse, avec de 0,9 à 1,5 mol de benzaldéhyde par mole d'acide 3,4-diaminobenzènesulfonique, et avec de 1,0 à 3,0 mol de SO₂ par mole d'acide 3,4-diaminobenzènesulfonique, ou un agent qui comprend de 1,0 à 3,0 mol de SO₂ par mole d'acide 3,4-diaminobenzènesulfonique.

2. Procédé selon la revendication 1, **caractérisé en ce que** l'on utilise l'acide 3,4-diaminobenzènesulfonique obtenu par sulfatation du 1,2-diaminobenzène.

3. Procédé selon l'une quelconque des revendications 1 à 2, **caractérisé en ce que** la réaction est effectuée à un pH dans la plage de 5 à 7.

4. Procédé selon l'une quelconque des revendications 1 à 3, **caractérisé en ce que** l'on utilise du benzaldéhyde en une quantité de 1,05 à 1,1 mol/mol.

5. Procédé selon l'une quelconque des revendications 1 à 4, **caractérisé en ce que** la réaction est effectuée à une température dans la plage de 25° à 130°C.
